# FASCICULE DE BREVET EUROPEEN

(11) **EP 0 666 764 B1**
(45) Date de publication et mention de la délivrance du brevet: **10.02.1999**
(21) Numéro de dépôt: 93924651.8
(22) Date de dépôt: 28.10.1993
(51) Int. Cl.: A61L 27/00, B30B 11/02

(54) **PROCEDE DE FABRICATION DE MATERIAUX, D'INTERET BIOLOGIQUE SIMPLES OU MULTIPHASES**
VERFAHREN ZUR HERSTELLUNG VON EIN-ODER MEHRPHASIGEN SUBSTANZEN VON BIOLOGISCHEM INTERESSE
METHOD FOR PRODUCING SINGLE- OR MULTI-PHASE BIOLOGICAL MATERIALS

(30) Priorité: 28.10.1992 FR 9212837
(43) Date de publication de la demande: 16.08.1995
(73) Titulaire: UNIVERSITE DE NANTES, F-44035 Nantes Cedex (FR)
(72) Inventeur: DACULSI, Guy, F-44460 Vigneux-de-Bretagne (FR); DEUDON, Catherine, F-44100 Nantes (FR)
(74) Mandataire: Dawidowicz, Armand
(86) Numéro de dépôt international: FR9301064
(87) Numéro de publication internationale: WO9409832

(56) Documents cités:
- EP-A- 0 087 662
- DE-A- 2 138 388
- DE-A- 3 706 821
- FR-A- 2 396 613
- US-A- 4 218 255

## Description

La présente invention concerne un procédé de compactage de phosphates de calcium purs ou composites, ainsi que les produits à usage biologique obtenus par la mise en oeuvre du procédé.

Les poudres minérales entrent aujourd'hui dans la composition d'un grand nombre de produits. Le problème essentiel réside toujours dans la liaison des éléments constitutifs desdites poudres entre eux. Ainsi, les céramiques constituées par des poudres minérales compactées ne sont obtenues que sous l'action soit d'une forte température et/ou d'une pression très élevée, provoquant de ce fait une détérioration des structures.

Parallèlement, la compaction dynamique est un procédé aujourd'hui largement utilisé en tant que procédé de soudage ou de liaison de poudres métalliques. Un tel procédé est par exemple étudié dans l'article de D.G. MORRIS "Bonding processes during the dynamic compaction of metallic powders" Mater .Sci.&Eng Vol 157 PP 187 à 195. De même, dans le brevet DE-A-2.138.388 décrit un procédé de compaction dynamique qui s'applique au compactage de poudres essentiellement métalliques. Le brevet FR-A-2.396.613 décrit quant à lui un procédé de compactage de poudre au moyen d'une onde de choc générée par lancement d'un projectile qui frappe directement la matrice à l'intérieur de la cavité des échantillons contenant la poudre. Ce brevet s'applique là encore aux poudres métalliques ou non sans précision supplémentaire.

Enfin, le brevet DE-A-3.706.821 décrit un procédé de compactage par explosion en vue de la fabrication de pièces céramiques réfractaires. Cependant, dans le cas de tous ces brevets, il n'est jamais cité d'application particulière des poudres obtenues.

Le principe de la compaction dynamique est simple et consiste à générer une onde de choc encore appelée onde de compression élastique qui percute l'échantillon placé dans une matrice de manière à le déformer en entraînant une fusion partielle des grains en surface et donc une liaison des grains entre eux.

Les inventeurs de la présente invention ont quant à eux découvert que, de manière surprenante, on pouvait obtenir sur des phosphates de calcium purs ou composites, le même résultat et obtenir ainsi sous forme solide des blocs de matériaux qui n'avaient jamais pu être compactés avec les techniques couramment utilisées telles que le frittage. Outre la possibilité d'obtenir de nouveaux matériaux, on évite également les inconvénients liés au frittage qui nécessite de comprimer le matériau à haute température de manière à entrainer une fusion des constituants puis une agglomération des microcristaux qui se forment au refroidissement, ceux-ci restant ensuite liés entre eux par des joints de grain. Cette élévation de la température modifie les propriétés cristallographiques et donc généralement l'activité biologique du matériau. De plus avec les poudres de phosphates de calcium, on obtient des céramiques en phosphates de calcium très fragiles car les échanges thermiques en particulier lors de l'étape de refroidissement du procédé de frittage entraînent des contraintes qui tendent à fissurer le produit fini.

Le but de la présente invention est donc de pouvoir réaliser, à partir de poudres de phosphates de calcium, des céramiques, voire des composites par association à des matrices métalliques ou non métalliques, sans élévation de température et avec des possibilités de contrôler la taille et la forme des cristaux finaux ainsi que l'homogénéité du mélange obtenu tout en conservant une activité biologique optimale de la composition.

Un autre but de la présente invention est de pouvoir associer des substances organiques, en particulier des principes actifs sans dégradation de ces derniers, à ces poudres de phosphates de calcium pour obtenir un relargage progressif dudit principe dans l'organisme.

Enfin, un dernier but de l'invention est d'obtenir de nouvelles céramiques à base de phosphates de calcium jamais fabriquées à ce jour.

L'invention concerne à cet effet un procédé de compactage de phosphates de calcium purs ou composites, caractérisé en ce qu'on prépare un échantillon contenant au moins un phosphate de calcium pulvérulent pur ou un mélange d'au moins un phosphate de calcium pulvérulent avec un produit ayant une activité biologique et en ce qu'on compacte dynamiquement directement ou indirectement ledit échantillon par une onde de choc de compression élastique.

L'invention concerne également un produit biocompatible à base d'au moins une poudre de phosphates de calcium dont les grains sont soudés entre eux à froid au moyen d'une onde de choc de compression élastique et conservent de ce fait la taille et la forme originelles des cristaux, telle qu'une forme allongée des cristaux.

D'autres caractéristiques et avantages de l'invention apparaîtront encore à la lecture de la description qui suit et des dessins joints, lesquels description et dessins sont donnés surtout à titre d'exemples. Dans ces dessins :
La figure 1 représente une vue en coupe d'une matrice de compactage; et
la figure 2 représente une vue schématique simplifiée d'un dispositif permettant le compactage dynamique de poudres.

Le procédé objet de l'invention permet la réalisation de produits bioactifs et de ce fait biocompatibles à partir de poudres de phosphate de calcium. Les produits obtenus sont constitués par des produits à base céramique associés ou non à d'autres éléments tels que du métal ou une résine ou des polymères, etc. selon qu'on veut réaliser une céramique en poudre minérale pure ou un matériau composite. Ces produits sont destinés par exemple à la réalisation d'implants osseux ou cartilagineux, à la réalisation de produits de comblement dégradables, etc.

Pour obtenir un tel résultat, il est nécessaire d'introduire un échantillon comprenant notamment une poudre minérale bioactive, c'est-à-dire une poudre minérale dont les constituants ont une activité biologique qui peut constituer en une modification de l'activité cellulaire, en une activité sur la cicatrisation, en une expression du phénotype de la cellule, etc. par le biais des échanges d'ions entre lesdits éléments constitutifs. Au moins l'un des éléments de cette poudre est choisi dans le groupe des phosphates de calcium comprenant les phosphates octocalciques, les phosphates dicalciques, les phosphates dicalciques hydratés, les phosphates tricalciques et les phosphates tétracalciques. Dans une deuxième étape, on génère une onde de choc appelée encore compression élastique qui va déformer l'échantillon et donc le densifier de manière à le compacter. L'onde de choc qui déforme l'échantillon et permet son compactage peut être générée de plusieurs manières.

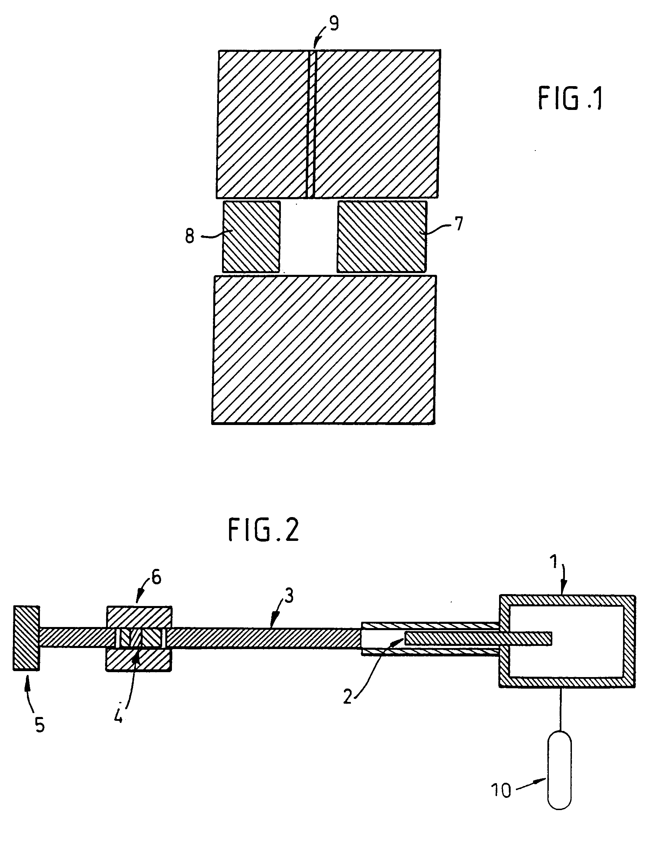

Un exemple d'un dispositif permettant la mise en oeuvre de ce procédé est représenté à la figure 1. Ce dispositif comprend des moyens de générer une onde de choc qui sont ici constitués par un lanceur à gaz désigné par 1 associé à un projectile. Le projectile désigné par 2 est propulsé par la pression du lanceur et vient heurter la barre d'incidence 3 en créant ainsi une onde de compression élastique. Cette barre vient percuter l'échantillon 4 placé dans la matrice créant ainsi une déformation et donc une densification des poudres a compacter. Lorsque l'onde atteint l'échantillon, une partie est réfléchie dans la barre d'incidence 3 et l'autre partie est transmise dans l'échantillon. L'amortissement est assuré par un absorbeur de choc constitué par une barre de transmission 4. On parlera dans l'exemple représenté à la figure 1 d'un compactage indirect dans la mesure où le choc a lieu sur la barre d'incidence 3 et est transmis par ladite barre 3 à l'échantillon.

On peut également imaginer que le choc ait lieu directement au niveau de l'échantillon, le projectile venant directement heurter l'échantillon ou les parois de la matrice. On peut également imaginer, dans un autre mode de réalisation de l'invention, de remplacer le lanceur à gaz et le projectile par un explosif qui va entraîner la génération d'une onde de choc agissant soit directement sur les parois de la matrice et/ou de l'échantillon soit indirectement par l'intermédiaire de moyens divers de transmission de l'onde. On parlera là encore dans ce cas d'un compactage direct ou indirect.

La vitesse de propulsion du projectile fonction de la composition des poudres peut varier. Elle est généralement de l'ordre de 60 m/s avec un canon chargé sous une pression de 15 bars. Les barres de Hopkinson utilisées, à savoir la barre d'incidence 3 et la barre de transmission 4 sont des barres d'acier qui résistent très bien à la contrainte transmise lors du choc par le projectile. La matrice utilisée pour contenir l'échantillon se compose généralement d'un cylindre ouvert à ses deux extrémités. Cette matrice constitue bien évidemment la chambre de compactage et peut constituer en outre une cavité de moulage. Dans ce cas, les produits obtenus n'ont pas besoin d'être usinés après compactage. Au contraire, il peut arriver de réaliser à l'intérieur de cette matrice un bloc de matériau, le bloc étant ensuite usiné pour obtenir la forme désirée.

Les poudres â compacter sont généralement retenues à l'intérieur de la matrice par deux pastilles fermant les extrémités du cylindre, comme le montre la figure 1 dans laquelle la poudre à compacter est disposée entre une pastille de compactage 7 et une contre-pastille 8. Pour éviter la formation d'un coussin d'air qui est néfaste pour le compactage et pour éviter toute contamination des poudres, la chambre contenant les poudres est mise sous vide secondaire ; le vide est de l'ordre de 3 x 10⁻² Pa. Le système de pompage du vide est connecté à un canal de pompage 9 du vide de la chambre cylindrique des poudres. Pour réaliser l'étanchéité à l'aspiration de la pompe avec l'extérieur, on dispose des membranes d'aluminium entre les barres et les pastilles, fixées aux extrémités du cylindre.

Bien évidemment pour que la barre d'incidence et la barre de transmission jouent pleinement leur rôle, il est prévu des bagues de guidage qui assument le centrage desdites barres. Selon les matériaux utilisés et les mélanges en jeu, les pressions varient. On a utilisé à titre d'exemple des pressions de l'ordre de 15 bars pour compacter des poudres à base de phosphate de calcium de formulation différente. Par contre des tirs ont été effectués à 40 bars pour compacter des composites métal/phosphate de calcium. De même, ces pressions ont été utilisées pour compacter des céramiques à base de phosphate de calcium ayant des cristaux de grande surface spécifique impossibles à réaliser par des méthodes classiques.

Grâce à la mise en oeuvre de ce procédé, on obtient des céramiques en phosphate de calcium ou des produits composites qui permettent d'obtenir une meilleure ostéo-intégration notamment lorsqu'ils constituent des implants, en raison du fait que ce procédé permet de mieux maîtriser les facteurs physico-chimiques tels que la température de préparation desdits céramiques qui agit sur la porosité et sur la taille des cristaux du matériau obtenu. En outre, il est possible d'envisager l'association d'un tel produit à base de poudres minérales, en particulier de poudre de phosphate de calcium, a un principe actif qui peut être un facteur de croissance, un antibiotique, etc., ou à toute autre substance organique active. Ce produit peut être intégré sous forme pulvérulente ou liquide avant compactage dans la mesure où le procédé n'inclut pas de montée en température. On obtient ainsi notamment lorsqu'on utilise le produit à base de poudre minérale comme implant, la possibilité d'un relargage progressif du principe actif.

De même, il est possible, grâce à ce procédé, d'obtenir de nouveaux produits que ce soit des produits composites ou de simples céramiques, à partir d'éléments choisis dans le groupe comprenant les phosphates octocalciques, les phosphates dicalciques, les phosphates dicalciques dihydratés, etc... On obtient ainsi de nouveaux biomatériaux dont les propriétés se révèlent particulièrement intéressantes. Ainsi, le procédé permet de conserver au cours de la compaction la taille et la forme des cristaux originelles. Par conséquent, ces matériaux ressemblant à des céramiques sont parfaitement identifiables en microscopie électronique à balayage et en microscopie à transmission du fait de la taille et de la forme des cristaux obtenus. En effet, les cristaux allongés sont toujours obtenus à basse température. A haute température, généralement, les cristaux se présentent sous forme de petites billes. Il est donc parfaitement aisé d'identifier si une céramique obtenue a nécessité la mise en oeuvre du procédé ou pas.

L'invention ne se limite pas aux matériaux biocompatibles à base de phosphates de calcium mais inclut au contraire tout matériau obtenu par compactage dynamique d'une poudre contenant des phosphates de calcium, avec ou sans principe actif, que ce dernier soit un agent d'adhésion, de chimiotachisme, un antibiotique, un antimitotique, une hormone ou un facteur de croissance.

## Revendications

1. Procédé de compactage de phosphates de calcium purs ou composites,
caractérisé en ce qu'on prépare un échantillon contenant au moins un phosphate de calcium pulvérulent pur ou un mélange d'au moins un phosphate de calcium pulvérulent avec un produit ayant une activité biologique et en ce qu'on compacte dynamiquement directement ou indirectement ledit échantillon par une onde de choc de compression élastique.

2. Procédé de compactage de phosphates de calcium purs ou composites selon la revendication 1,
caractérisé en ce qu'on génère l'onde de choc de compression élastique par explosion.

3. Procédé de compactage de phosphates de calcium purs ou composites selon la revendication 1,
caractérisé en ce qu'on génère l'onde de choc de compression élastique par lancement d'un projectile qui heurte directement ou non une matrice (6) à l'intérieur de laquelle est disposé l'échantillon.

4. Procédé de compactage de phosphates de calcium purs ou composites selon la revendications 3,
caractérisé en ce que la matrice formant chambre de compactage qui contient l'échantillon constitue une cavité de moulage.

5. Procédé de compactage de phosphates de calcium purs ou composites selon la revendication 4,
caractérisé en ce que la chambre de compactage est mise sous vide.

6. Produit, d'intérêt biologique, tel qu'implant osseux ou cartilagineux, médicament, à base d'au moins une poudre de phosphates de calcium purs ou composites compactée,
caractérisé en ce que les grains de la poudre de phosphates de calcium purs ou composites sont soudés entre eux à froid au moyen d'une onde de choc de compression élastique et conservent de ce fait la taille et la forme originelles des cristaux telle qu'une forme allongée des cristaux.

## Claims

1. Compaction process for calcium phosphates either pure or compound,
characterised in that a sample is prepared containing at least a pulverulent pure calcium phosphate or a mixture of at least a pulverulent calcium phosphate with a product having a biological strength and in that compaction of the said sample is made dynamically either directly or indirectly with an elastic compression shock wave.

2. Compaction process for calcium phosphates either pure or compound according to claim 1,
characterised in that the elastic compression shock wave is produced by an explosion.

3. Compaction process for calcium phosphates either pure or compound according to claim 1,
characterised in that the elastic compression shock wave is produced by throwing a projectile which strikes directly or not a die (6) inside which the sample has been placed.

4. Compaction process for calcium phosphates either pure or compound according to claim 3,
characterised in that the die which forms the compaction chamber containing the sample constitutes a moulding cavity.

5. Compaction process for calcium phosphates either pure or compound according to claim 4,
characterised in that the compaction chamber is in vacuum.

6. Product, of biological interest, such as a bony or cartilaginous implant, medicine, based at least on a compacted powder of calcium phosphate either pure or compound,
characterised in that the calcium phosphate powder grains either pure or compound are cold welded between themselves by means of an elastic compression shock wave and retain thus the original size and shape of the crystals such as an elongated shape of the crystals.

## Patentansprüche

1. Verfahren zur Verdichtung von reinen oder zusammengesetzten Kalziumphosphaten,
dadurch gekennzeichnet, daß eine Probe vorbereitet wird, die wenigstens ein pulverförmiges reines Kalziumphosphat oder eine Mischung wenigstens eines pulverförmigen Kalziumphosphates mit einem Produkt, das eine biologische Aktivität aufweist, enthält, vorbereitet wird und daß die genannte Probe dynamisch direkt oder indirekt durch eine elastische Verdichtungsstoßwelle verdichtet wird.

2. Verfahren nach Anspruch 1 zur Verdichtung von reinen oder zusammengesetzten Kalziumphosphaten,
dadurch gekennzeichnet, daß die elastische Verdichtungsstoßwelle durch eine Explosion erzeugt wird.

3. Verfahren nach Anspruch 1 zur Verdichtung von reinen oder zusammengesetzten Kalziumphosphaten,
dadurch gekennzeichnet, daß die elastische Verdichtungsstoßwelle durch Abschuß eines Projektils erzeugt wird, das direkt oder nicht direkt auf eine Matrize (6) auftrifft, in deren Innerem die Probe angeordnet ist.

4. Verfahren nach Anspruch 3 zur Verdichtung von reinen oder zusammengesetzten Kalziumphosphaten,
dadurch gekennzeichnet, daß die Matrize, die eine Verdichtungskammer bildet, die die Probe enthält, einen Gießhohlraum bildet.

5. Verfahren nach Anspruch 4 zur Verdichtung von reinen oder zusammengesetzten Kalziumphosphaten,
dadurch gekennzeichnet, daß die Verdichtungskammer mit Unterdruck beaufschlagt wird.

6. Produkt von biologischem Interesse, beispielsweise ein Knochen- oder Knorpelimplantat oder ein Medikament auf Basis wenigstens eines verdichteten Pulvers aus reinen oder zusammengesetzten Kalziumphosphaten,
dadurch gekennzeichnet, daß die Körper des Pulvers aus reinem oder zusammengesetztem Kalziumphosphat miteinander mittels einer elastischen Verdichtungsstoßwelle kaltverschweißt sind und aus diesem Grund die ursprüngliche Größe und Form der Kristalle bewahren, beispielsweise eine gestreckte Form der Kristalle.
